# EUROPÄISCHE PATENTSCHRIFT

(11) **EP 1 509 147 B1**
(45) Veröffentlichungstag und Bekanntmachung des Hinweises auf die Patenterteilung: **15.08.2007**
(21) Anmeldenummer: 03727236.6
(22) Anmeldetag: 19.05.2003
(51) Int. Cl.: A61B 17/70

(54) **VERANKERUNGSELEMENT ZUR BEFESTIGUNG EINES STABES AN EINEM WIRBELKNOCHEN**
ANCHORING ELEMENT FOR SECURING A ROD ON A VERTEBRA
ELEMENT D'ANCRAGE SERVANT A FIXER UNE TIGE SUR UNE VERTEBRE

(30) Priorität: 21.05.2002 DE 20207852 U; 21.05.2002 DE 20207850 U
(43) Veröffentlichungstag der Anmeldung: 02.03.2005
(60) Teilanmeldung: 07006816.8
(73) Patentinhaber: Metz-Stavenhagen, Peter, 34537 Bad Wildungen (DE)
(72) Erfinder: Metz-Stavenhagen, Peter, 34537 Bad Wildungen (DE)
(74) Vertreter: WALTHER, WALTHER & HINZ Patentanwälte - European Patent Attorneys
(86) Internationale Anmeldenummer: PCT/DE2003/001610
(87) Internationale Veröffentlichungsnummer: WO 2003/096916

(56) Entgegenhaltungen:
- EP-A- 1 064 885
- EP-A- 1 190 678
- WO-A-95/01132
- US-B1- 6 251 112
- US-B1- 6 258 090

## Beschreibung

Die vorliegende Erfindung betrifft ein Verankerungselement zur Befestigung eines Stabes einer Vorrichtung zum Einrichten einer menschlichen oder tierischen Wirbelsäule an einem Wirbelknochen gemäß dem Oberbegriff des Anspruches 1.

Aus dem DE 94 02 839 U ist eine Pedikelschraube bekannt, die mit ihrem Gewindeschaft in einem Wirbelkörper verankerbar ist und die an ihrem aus dem Wirbelknochen herausragenden Ende eine U-förmig gebildete Halterung mit zwei parallel ausgerichteten Haltestegen aufweist. Dabei ist zwischen den Haltestegen ein Aufnahmeschlitz zur Aufnahme eines Distraktions- oder Kompressionsstabes ausgebildet, der mittels einem an den freien Enden der Haltestege befestigbaren Sicherungselement gesichert und fixiert werden kann. Dieses Sicherungselement setzt sich in einer ersten Ausführungsform aus einem sechseckigen, ringförmigen Element mit einer darin eingelassenen Feststellschraube zusammen, wobei das gesamte Sicherungselement in eine entsprechende Nut in den Haltestegen einschiebbar ist. Das Sicherungselement überspannt den Aufnahmeschlitz, so dass die Feststellschraube direkt auf den Stab wirkt, um diesen zu fixieren.

In einer anderen Ausführungsform setzt sich das Sicherungselement aus einer das Halteelement umschließenden Hülle mit Innengewinde zusammen, wobei die Hülle auf ein entsprechendes, auf den Außenseiten der Haltestege vorgesehenes Gewinde aufschraubbar ist, so dass die auf der Stirnseite der Hülle vorgesehene Feststellschraube in den Aufnahmeschlitz eingreifen kann, um den Stab zu fixieren.

Bei einer derartigen Vorrichtung muss also zunächst einmal die Pedikelschraube im Wirbelkörper verankert werden, um anschließend den Stab in den Aufnahmeschlitz einzulegen. Danach wird das Sicherungselement entweder in die Nuten eingeführt oder aber auf die Halterung aufgeschraubt, bevor der Wirbel bzw. der Stab durch Anziehen der Feststellschraube in die gewünschte Position gebracht wird. Dies hat sich in der Praxis als sehr schwierig herausgestellt, da entweder das Einführen des Sicherungselementes in die Nuten oder das Aufschrauben des Sicherungselementes auf die Halterung sehr viel Fingerspitzengefühl erfordert, wobei gleichzeitig darauf geachtet werden muss, dass der Wirbelkörper und/oder der Stab währenddessen nicht verrutscht.

Aus dem DE 297 10 484 U ist ein Verankerungselement bekannt, bei dem an den Innenseiten der Haltestege ein Sägezahngewinde zur Aufnahme der Feststellschraube vorgesehen ist. Hierdurch wird erreicht, dass die bei einem normalen Gewinde radial.auf die Haltestege wirkenden Kräfte entfallen und lediglich axial wirkende Kräfte am Haltesteg angreifen. Hierdurch wird ein Auseinanderbiegen der Haltestege verhindert, so dass auch das die Haltestege umfassende Ringelement entfallen kann. Auch hier hat es sich in der Praxis als sehr mühsam herausgestellt, die jeweilige Feststellschraube in das entsprechende Sägezahngewinde einzuschrauben und dabei gleichzeitig die Wirbelsäule und/oder den Stab so lange in der gewünschten Position zu halten, bis die Feststellschraube den Stab selbständig hält.

Unter einem Sägezahngewinde sind neben dem metrischen Sägengewinde gemäß DIN 513 auch Sägezahngewinde mit einem etwa größeren oder etwas kleineren Flankenwinkel, mit einem Flankenwinkel von 0° oder einem negativen Flankenwinkel, sowie Sägezahngewinde gemäß EP 885 598 zu verstehen.

Aus der WO 95/01132 ist ein Verankerungselement bekannt, welches ebenfalls eine Pedikelschraube mit daran angeformten U-förmigen Haltestegen aufweist. An diesen Haltestegen sind ins Innere des U gerichtete Pins angeformt. Des Weiteren umfasst dieses Verankerungselement ein flanschförmiges Sicherungselement, an dessen Oberseite ein umlaufender und überstehender - Kragen ausgebildet ist und an dessen Unterseite zwei gewinkelte Nuten zum Einsatz dieses Sicherungselementes in die Pins der Halteelemente vorgesehen ist. Des Weiteren umfasst das Sicherungselement ein Innengewinde, in welches eine Madenschraube zur Fixierung des Stabes eindrehbar ist.

Bei der Operation wird nun zunächst die Pedikelschraube an der betreffenden Stelle im Knochen eingesetzt, bevor der Fixierstab in die U-förmige Halterung gebracht wird. Aufgrund des wenigen, im Inneren des U-förmigen Halters zur Verfügung stehenden Platzes kann der Pin an der Innenseite der Haltestege nur sehr klein ausgelegt werden. Aus diesem Grunde ist es nach Einlegen des Fixierstabes für den behandelnden Arzt sehr schwer, das Sicherungselement anschließend zielgenau auf diese Pins aufzusetzen und zu verschwenken. Erschwert wird diese Tätigkeit noch dadurch, dass beim Einsetzen des Sicherungselementes in den U-förmigen Halter kein Sichtkontakt zum Pin besteht, da dieser versteckt ist. Auch hier wird der Fixierstab zunächst grob durch das Sicherungselement gehalten, um anschließend von der Madenschraube in seiner endgültigen Position fixiert zu werden.

Aus dem DE 92 02 745 U1 ist eine Pedikelschraube bekannt, die mit ihrem Gewindeschaft in einem Wirbelkörper verankerbar ist und die an ihrem aus dem Wirbelknochen herausragenden Ende eine U-förmig gebildete Halterung mit zwei parallel ausgerichteten Haltestegen aufweist. Dabei ist zwischen den Haltestegen ein Aufnahmeschlitz zur Aufnahme eines Distraktions- oder Kompressionsstabes ausgebildet, der mittels einem an den freien Enden der Haltestege befestigbaren Sicherungselement gesichert und fixiert werden kann. Dieses Sicherungselement ist als Madenschraube ausgebildet und greift in entsprechende, an den jeweils innenliegenden Seiten der Haltestege angeordnete Gewindeabschnitte ein. Zur Betätigung der Madenschraube ist an ihrer oberen Stirnseite eine Aufnahme für einen Sechskantschlüssel ausgebildet.

Aus der US 6,224,598 B1 ist ein Verankerungselement zur Befestigung eines Stabes an einer Wirbelsäule offenbart, welches sich ebenfalls aus einer Pedikelschraube, einer daran angeformten U-förmigen Halterung und einem Verschlusselement zusammensetzt. Hier ist das Verschlusselement zweiteilig ausgebildet, wobei ein äußeres Teil als Hülse sowohl mit einem Innen-, als auch mit einem Außengewinde ausgebildet ist, und wobei ein inneres Teil bolzenartig geformt ist, und an einem Ende ein Gewinde und am anderen Ende ein Sechskant aufweist. Hierbei wird die Hülse mit den Fingern in das Gewinde des Halterelementes eingeschraubt und hält den Fixierstab zunächst einmal provisorisch. Danach wird der Bolzen in die Hülse eingeschraubt und so tief eingedreht, dass das Gewinde des Bolzens vollständig in der Hülse verschwunden ist. Wird der Bolzen weiter gedreht, so nimmt dieser die Hülse mit und beide Teile bewegen sich auf den Fixierstab zu, um diesen zuverlässig festzusetzen.

Aus der EP 1 064 885 A1 (Basis für den Oberbegriff des Anspruchs 1) ist eine Haltevorrichtung für eine Wirbelsäule bekannt, bei der auf ein hakenförmiges Halteelement eine Fixierstange aufgebracht wird, die wiederum von zwei U-förmigen Stegen umfasst ist. In diesen Stegen sind in Richtung der Längsachse verlaufende Schlitze eingelassen, wobei am unteren Ende der Schlitze quer zur Längsachse verlaufende Aussparungen vorgesehen sind. Auf dieses Halteelement und auf die Fixierstange wird ein Deckel mit zwei seitlich angebrachten Flanken aufgesetzt, wobei an den Flanken nach innen gerichtete Stifte angebracht sind, die in die Schlitze des Halteelementes eingreifen sollen. Diese Stifte sind zwar quer zur Längsachse ausgerichtet, jedoch nicht radial, sondern in einem bestimmten Winkel zur radialen Richtung angeordnet.

Bei derartigen Vorrichtungen muss zunächst einmal die Pedikelschraube in den Wirbelkörper eingeschraubt werden, um anschließend den Stab in den Aufnahmeschlitz einzulegen. Danach wird die Madenschraube in die Halterung eingeschraubt, um den Stab lose zu halten. Anschließend wird der Wirbelkörper durch Justieren des Stabes in die gewünschte Position gebracht, bevor die Madenschraube fest angezogen wird, um den Stab in dieser Position zu fixieren. Dabei kann der Stab beispielsweise als Distraktions-, Kompressions- oder Verbindungsstab ausgebildet sein.

Davon ausgehend liegt der vorliegenden Erfindung die Aufgabe zugrunde, ein Verankerungselement zu schaffen, bei dem das Sicherungselement die auftretenden Kräfte zuverlässig aufnimmt und bei dem das Sicherungselement einfach und schnell anzubringen ist.

Als technische Lösung dieser Aufgabe wird erfindungsgemäß ein Verankerungselement mit dem Merkmal des Anspruches 1 vorgeschlagen. Vorteilhafte Weiterbildungen sind den Unteransprüchen zu entnehmen.

Ein nach dieser technischen Lehre ausgebildetes Verankerungselement hat den Vorteil, dass das Ringelement lediglich axial auf die freien Ende der Haltestege aufgesetzt werden braucht und durch ein leichtes Verschwenken in seine endgültige, gesicherte Position gebracht werden kann. Ein derart gesichertes Ringelement hält den im Aufnahmeschlitz befindlichen Stab bereits provisorisch in der gewünschten Position, so dass der operierende Arzt nun in Ruhe das Feststellelement einschrauben kann, um den Stab zu justieren und zu fixieren.

Dabei hat es sich als vorteilhaft erwiesen, die Renkverbindung als Bajonettverschluss, mit einem radial abstehenden Stift und einer schlitz- oder nutartigen Aufnahme auszubilden, wobei der Stift in die Aufnahme eingreift, um das Ringelement der Haltestege zu fixieren. Ein solcher Bajonettverschluss ist kostengünstig realisierbar und erlaubt eine schnelle und einfache Anbringung des Ringelementes an der Halterung des Verankerungselementes.

Ein weiterer Vorteil besteht darin, dass der Stift durch die Aufnahme hindurch und aus dieser herausreicht und derart gekrümmt oder geneigt ist, vorzugsweise in Richtung der auf das Sicherungselement weilenden Kräfte, dass der Haltesteg nach innen gedrückt wird. Hierdurch werden die Haltestege aufgrund der wirkenden Haltekräfte des Verankerungselementes nach innen gedrückt und es ist gewährleistet, dass das Ringelement zuverlässig in den Haltestegen gehalten wird. Ein Herausspringen des Sicherungselementes aufgrund sich aufbiegenden Haltestegen wird so verhindert.

Die Ausgestaltung der Aufnahme mit einer axialen Einführung und einem tangentialen Halteelement hat den Vorteil, dass der Stift in einfacher Weise in die Einführung gebracht werden kann und durch Verdrehen in das Halteelement gelangt, um dort zu verharren und einen formschlüssigen Bajonettverschluss zu bilden.

Es versteht sich, dass der Stab beispielsweise als Distraktions-, Kompressions- oder Verbindungsstab ausgebildet sein kann.

In einer bevorzugten Ausführungsform ist das Ringelement in den Aufnahmeschlitz einführbar und zwischen den Haltestegen anbringbar, wobei der Stift außen am Ringelement angebracht ist, während die Aufnahme in den Haltestegen angeordnet ist. Dies hat den Vorteil, dass hiermit das Ringelement ebenfalls im Aufnahmeschlitz integriert ist, so dass das gesamte Verankerungselement sehr klein ausgeführt werden kann.

In noch einer anderen, bevorzugten Ausführungsform ist die Einführung V-förmig ausgebildet. Dies hat den Vorteil, dass der in diese Einführung einzuführende Stift lediglich grob auf die Einführung aufgesetzt werden braucht und anschließend durch die V-förmige Ausgestaltung dennoch korrekt bis zum quer ausgerichteten Halteelement geführt wird, um anschließend durch Verschwenken des Ringelementes in dieses eingeführt zu werden. Hierdurch wird das Aufsetzen des Ringelementes auf die Haltestege weiter vereinfacht.

In einer weiteren bevorzugten Ausführungsform ist das Halteelement nach oben ansteigend ausgebildet, wodurch das Ringelement nicht nur eine drehende, sondern zusätzlich eine axiale Bewegung vollziehen muss, um gelöst zu werden. Durch solch ein Zusatzerfordernis beim Lösen des Ringelementes wird ein unbeabsichtigtes Lösen des Ringelementes erschwert. Analoges gilt für eine Verbreiterung des Endes des Halteelementes.

Weitere Vorteile des erfindungsgemäßen Verankerungselementes ergeben sich aus der beigefügten Zeichnung und den nachfolgend beschriebenen Ausführungsformen. Ebenso können die vorstehend genannten und die noch weiter ausgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter. Es zeigen:
- Fig. 1: eine Explosionsdarstellung eines Verankerungselementes mit einem Stab;
- Fig. 2: eine Draufsicht auf das Ringelement gemäß Fig. 1;
- Fig. 3: eine teilweise geschnittene Seitenansicht des Verankerungselementes gemäß Fig. 1, geschnitten entlang Linie III - III in Fig. 1;
- Fig. 4: eine Explosionsdarstellung zweiten eines Verankerungselementes mit einem Stab;
- Fig. 5: eine Draufsicht auf das Ringelement gemäß Fig. 4;
- Fig. 6: eine Seitenansicht des Verankerungselementes gemäß Fig. 4;
- Fig. 7: eine Explosionsdarstellung dritten eines Verankerungselementes mit einem Stab;
- Fig. 8: eine Draufsicht auf das Ringelement gemäß Fig. 7;
- Fig. 9: eine teilweise geschnittene Seitenansicht des Verankerungselementes gemäß Fig. 7, geschnitten entlang Linie IX - IX in Fig. 7;
- Fig. 10: eine Explosionsdarstellung vierten eines Verankerungselementes mit einem Stab;
- Fig. 11: eine Draufsicht auf das Ringelement gemäß Fig. 10;
- Fig. 12: eine Seitenansicht des Verankerungselementes gemäß Fig. 10;
- Fig. 13: eine Explosionsdarstellung fünften eines Verankerungselementes mit einem Stab;
- Fig. 14: eine Draufsicht auf das Ringelement gemäß Fig. 13;
- Fig. 15: eine Seitenansicht des Verankerungselementes gemäß Fig. 13;
- Fig. 16: eine Explosionsdarstellung schesten eines Verankerungselementes mit einem Stab;
- Fig. 17: eine Draufsicht auf das Ringelement gemäß Fig. 16;
- Fig. 18: eine teilweise geschnittene Seitenansicht des Verankerungselementes gemäß Fig. 16, geschnitten entlang Linie XVIII - XVIII in Fig. 16;
- Fig. 19: einer Explosionsdarstellung einer Ausführungsform eines erfindungsgemäßen Verankerungselementes;
- Fig. 20: eine Draufsicht auf das Ringelement gemäß Fig. 19;
- Fig. 21: eine Seitenansicht des Verankerungselementes gemäß Fig. 19.
- Fig. 22: eine Explosionsdarstellung eines Verankerungselementes mit einem Stab;
- Fig. 23: eine Draufsicht auf die Halterung des Verankerungselementes gemäß Fig. 22;
- Fig. 24: eine Draufsicht auf das Sicherungselement des Verankerungselementes gemäß Fig. 22;
- Fig. 25: eine perspektivische Ansicht des Verankerungselementes gemäß Fig. 22, wobei das Sicherungselement explosionsartig herausgezogen ist;
- Fig. 26: eine perspektivische Ansicht des Verankerungselementes gemäß Fig. 27, wobei das Sicherungselement in die Halterung eingesetzt ist;
- Fig. 27: eine Ausschnittsvergrößerung des Verankerungselementes gemäß Fig. 23;
- Fig. 28: eine Explosionsdarstellung einer zweiten Ausführungsform eines erfindungsgemäßen Verankerungselementes mit einem Stab;
- Fig. 29: eine Draufsicht auf die Halterung des Verankerungselementes gemäß Fig. 28;
- Fig. 30: eine Draufsicht auf das Sicherungselement des Verankerungseiementes gemäß Fig. 28.

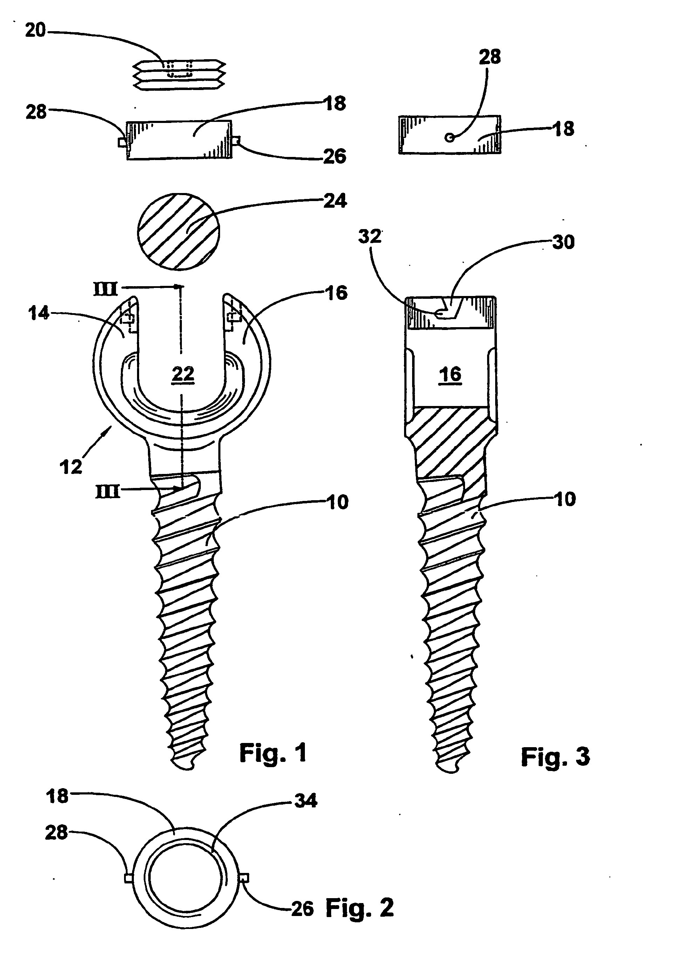

In den Figuren 1 bis 3 ist ein als Pedikelschraube ausgebildetes Verankerungselement dargestellt, wobei das Verankerungselement zur Einbringung in den Wirbelknochen einen Gewindeschaft 10, sowie eine U-förmig ausgebildete Halterung 12 mit zwei im Wesentlichen parallel angeordneten Haltestegen 14, 16, ein Ringelement 18 und ein als Madenschraube ausgeführtes Feststellelement 20 umfasst. Dabei ist in der Halterung 12 ein Aufnahmeschlitz 22 zur Aufnahme eines Stabes 24 vorgesehen, welcher über das sich aus dem Feststellelement 20 und dem Ringelement 18 zusammensetzenden Sicherungselement fixiert wird.

In dieser Schraube sind am Ringelement 18 an sich gegenüberliegenden Seiten zwei Bajonett-Stifte 26, 28 radial abstehend ausgebildet, welche in eine entsprechende, an der Innenseite der Haltestege 14,16 ausgebildete Einführung 30 und ein Halteelement 32 einsetzbar sind. Dabei wird das Ringelement 18 mit seinen beiden Stiften 26, 28 von oben in die nach oben offene Einführung 30 eingeführt, und bis an den Grund der Einführung 30 gebracht, bevor das Ringelement 18 um einige Winkelgrade verschwenkt wird, so dass die Stifte 26, 28 bis ans Ende des Halteelementes 32 geführt werden. In dieser Position angekommen, wird das Ringelement 18 losgelassen und verbleibt zuverlässig in dieser Position.

Wie aus Figur 3 gut ersichtlich ist, ist die Einführung 30 V-förmig ausgebildet, um einen großen Fangbereich zum Einführen der Stifte 26, 28 zu bilden.

Im Ringelement 18 ist ein Innengewinde 34 zur Aufnahme des als Madenschraube ausgebildeten Feststellelementes 20 vorgesehen. Dabei wird das Feststellelement 20 so weit in das Ringelement 18 hereingeschraubt, dass das Feststellelement 20 auf den Stab 24 drückt und diesen in der gewünschten Position fixiert.

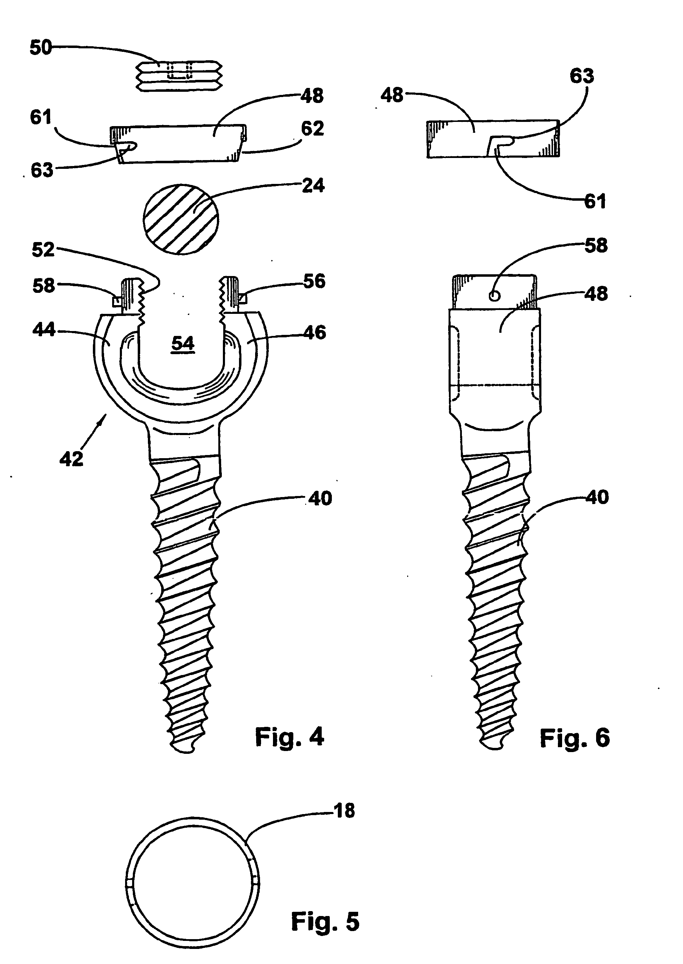

In den Fig. 4 bis 6 ist eine zweite als Pedikelschraube ausgeführten Verankerungselement dargestellt. Auch hier setzt sich das Verankerungselement aus einem Gewindeschaft 40 zur Anbringung des Verankerungselementes im Wirbelknochen, einer U-förmig ausgebildeten Halterung 42 mit zwei im Wesentlichen parallel angeordneten Haltestegen 44, 46, einem Ringelement 48 und einem als Madeschraube ausgeführten Feststellelement 50 zusammen. In dieser Schraube ist an den jeweiligen Innenseiten der Haltestege 44, 46 ein Innengewinde 52 zur Aufnahme des als Madenschraube ausgebildeten Feststellelementes 50 ausgebildet. Damit in dieser Schraube die auf die Haltestege 44, 46 wirkenden, radialen Kräfte nicht zu einem Aufbiegen der Haltestege 44, 46 führen, wird hierbei das Ringelement 48 außen um die Haltestege 44, 46 herumgelegt.

Auch in dieser Schraube ist in der Halterung 42 ein Aufnahmeschlitz 54 eines Stabes 24 ausgebildet, welcher durch das Feststellelement 50 in der gewünschten Position festgestellt wird.

An den nach außen weisenden Seiten der Haltestege 44, 46 ist jeweils ein radial abstehender Stift 56, 58 vorgesehen, die in entsprechende, innenseitig am Ringelement 48 vorgesehene Einführungen 61 einführbar sind, wobei sich auch hier an die Einführung 60, 61 quasi rechtwinklig entsprechende Halteelemente 62, 63 anschließen. Auch hier sind die Einführungen 60,61 V-förmig ausgeführt, um einen großen Fangbereich zu realisieren.

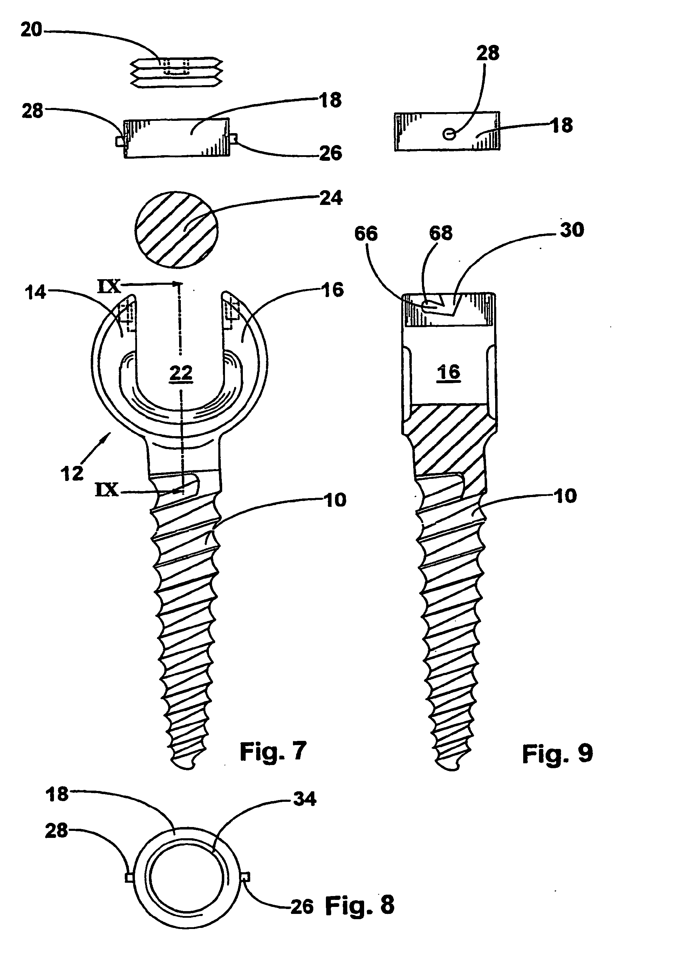

In den Fig. 7 bis 9 ist ein drittes, als Pedikelschraube ausgeführten Verankerungselement dargestellt. Diese dritte Schraube unterscheidet sich von der ersten Schraube gemäß Fig. 1 bis 3 dadurch, dass an der Innenseite der Haltestege 14, 16, sich an die Einführung 30 anschließend eine leicht nach oben ansteigendes Halteelement 66 ausgebildet ist, an dessen Ende eine Verbreiterung 68 vorgesehen ist. Dabei ist die Verbreiterung 68 im Wesentlichen kreisförmig gestaltet. In diesem Halteelement 66 ist der Stift 26, 28 einführbar und verklemmt sich bei entsprechendem Verdrehen des Ringelementes 18 in dem nach oben laufenden Halteelement 66, so dass hierdurch eine Klemmsicherung erreicht wird. Dabei steigt das Halteelement 66 in Richtung der auf das Sicherungselement wirkenden Kräfte.

Die Verbreiterung 68 am Ende des Halteelementes 66 bewirkt auch, dass die beim Verdrehen des Ringelementes 18 zwischen Stift 26, 28, bzw. Ringelement 18 einerseits und den Haltestegen 14, 16 andererseits aufgebaute Spannung zumindest teilweise wieder abgebaut wird, da sich die Verbreiterung 68 teilweise in Richtung der Haltekraft erstreckt und somit den Druck zwischen Sicherungselement und Stab 24 zumindest teilweise aufhebt. Gleichzeitig wird hierdurch eine zuverlässige und dauerhafte Sicherung des Ringelementes 18 erreicht, da zum Lösen des Ringelementes 18 eben dieser Druck wieder aufgebaut werden muss, was ohne externe, gezielte Krafteinwirkung nicht möglich ist. Dies bedeutet, dass sich das Ringelement 18 nicht von alleine lösen kann.

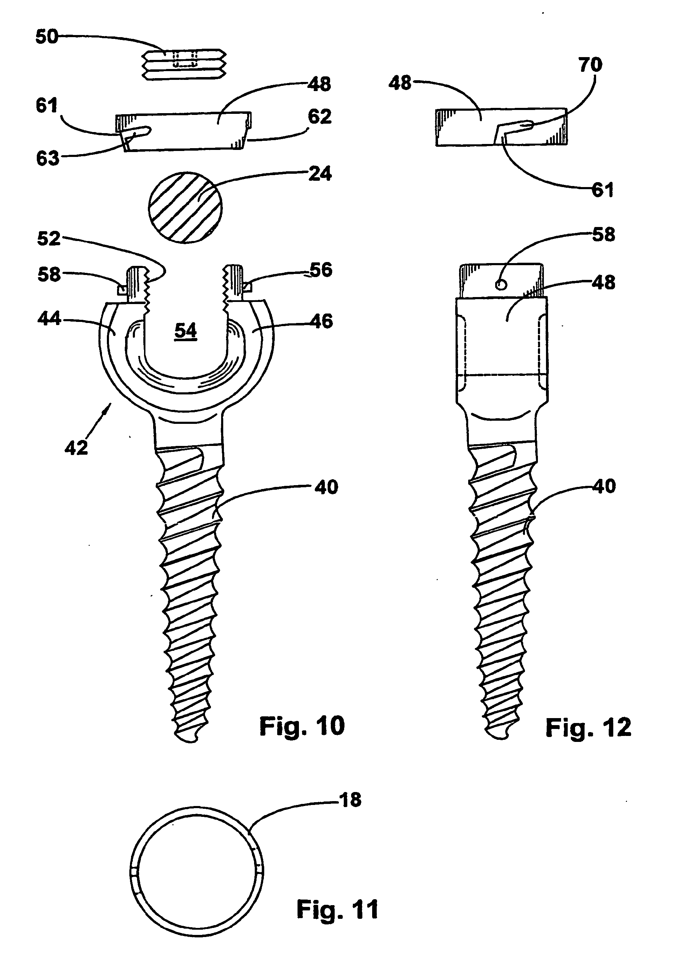

In den Fig. 10 bis 12 ist ein viertes als Pedikelschraube ausgeführten Verankerungselement dargestellt. Diese vierte Schraube unterscheidet sich von der zweiten Schraube gemäß Fig. 4 bis 6 dadurch, dass an der Innenseite des Ringelementes 48, sich an die Einführung 61 anschließend ein leicht nach oben ansteigendes Halteelement 70 ausgebildet ist. In dieses Halteelement 70 ist der Stift 56, 58 einführbar und verklemmt sich bei entsprechendem Verdrehen des Ringelementes 48 in dem nach oben laufenden Halteelement 70, so dass hierdurch eine Klemmsicherung erreicht wird.

In einer hier nicht dargestellten Ausführungsform ist das Ende des Halteelementes 70, analog zum Halteelement 66 gemäß Fig. 9, verbreitert ausgebildet.

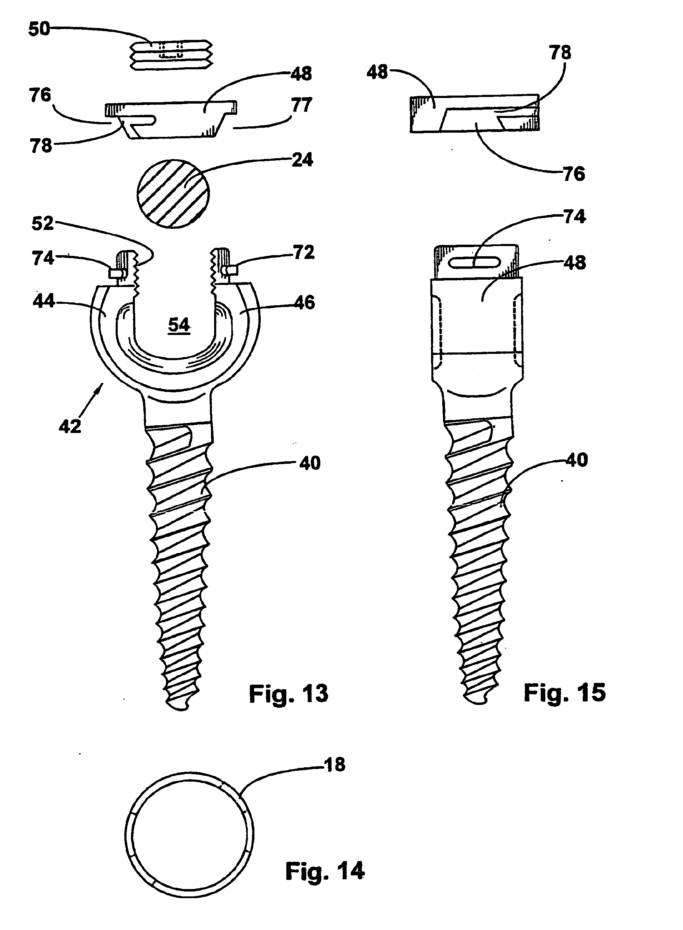

In den Fig. 13 bis 15 ist ein fünftes als Pedikelschraube ausgeführten Verankerungselement dargestellt. Diese fünfte Schraube unterscheidet sich von der zweiten Schraube gemäß Fig. 4 bis 6 dadurch, dass hier der Greifer als Bajonett-Flanke 72, 74 ausgebildet ist, die in eine entsprechende Einführung 76, 77 und Halteelement 78 am Ringelement 48 einführbar ist. Durch die breite Flanke 72, 74 entsteht eine große Anlagefläche zwischen Ringelement 48 und Haltesteg 44, 46, was zu einer großen Reibung führt, sobald das Ringelement 48 vorschriftsmäßig aufgesetzt ist. Diese Reibung wiederum erschwert das unbeabsichtigte Lösen des Ringelementes 48, so dass hierdurch eine Sicherung gegen unbeabsichtigtes Lösen des Ringelementes 48 erreicht wird.

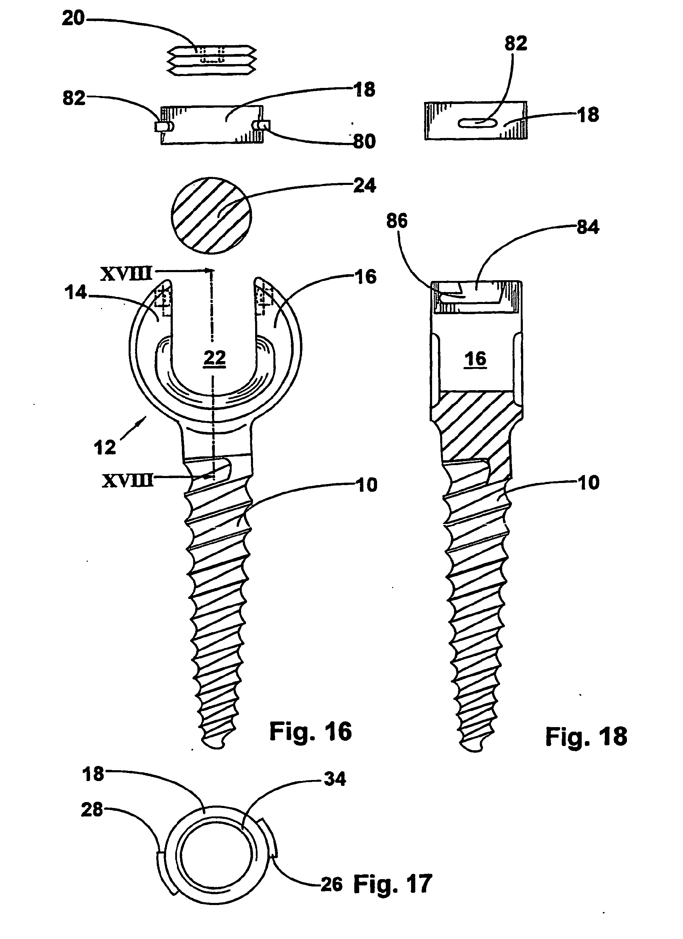

In den Fig. 16 bis 18 ist ein sechstes als Pedikelschraube ausgeführten Verankerungselement dargestellt. Diese sechste Schraube unterscheidet sich von der ersten Schraube gemäß Fig. 1 bis 3 dadurch, dass auch hier die Stifte durch Flanken 80, 82 ersetzt wurden, die in eine entsprechende Einführung 84 und Halteelement 86 eingreifen. Im Übrigen gilt das zur fünften Schraube Gesagte analog.

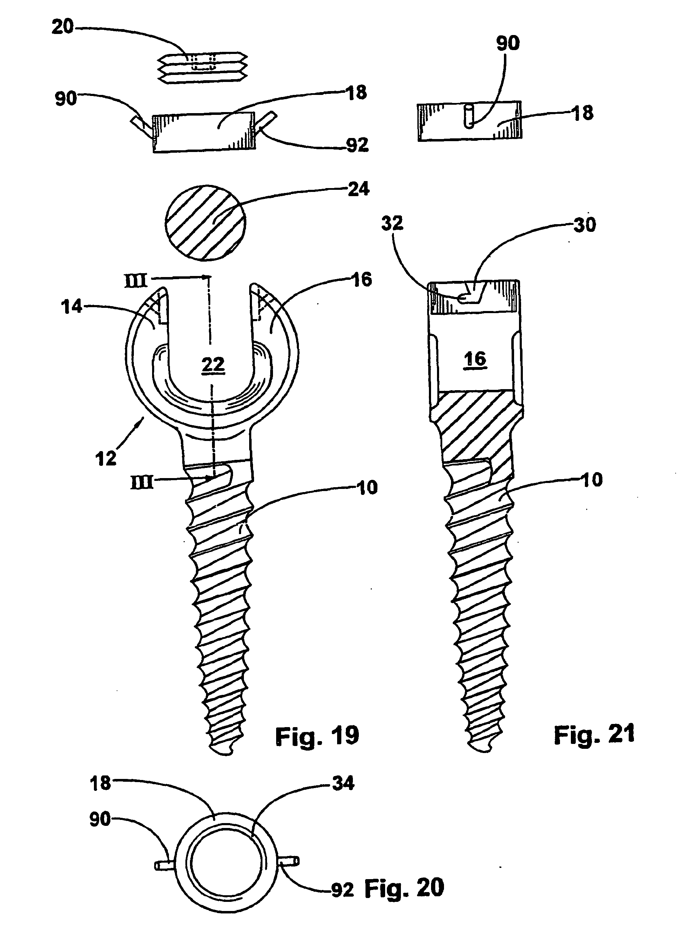

Die in den Fig. 19 bis 21 dargestellte Ausführungsform eines erfindungsgemäßen Verankerungselementes entspricht im wesentlichen der in den Fig. 1 bis 3 gezeigten ersten Schraube, einzig der Greifer des Bajonettverschlusses ist hier anders ausgebildet. Wie Fig. 19 zu entnehmen ist, ist der Greifer des Bajonettverschlusses als ein radial abstehender, nach oben geneigter Stift 90, 92 ausgebildet. Dieser Stift 90, 92 ist länger als der vergleichbare Stift 26, 28 in Fig. 1.

Nachdem der Stab 24 in den Aufnahmeschlitz 22 eingelegt wurde, wird das Ringelement 18 ebenfalls in axialer Richtung in den Aufnahmeschlitz 22 eingeführt. Dabei sind die Stifte 90, 92 so ausgerichtet, dass sie in die Einführung 30 gelangen. Nun muss der operierende Chirurg das Ringelement 18 mit angemessener Kraft auf den Stab 24 aufdrücken, so das letzterer tiefer in den Aufnahmeschlitz 22 hineingedrückt wird, und so dass die Stifte 90, 92 bis auf den Grund der Einführung 30 gelangen. In dieser Position angekommen, kann das Ringelement 18 um eine Winkelbreite verschwenkt werden, so dass die Stifte 90, 92 in das Halteelement 32 gelangen. Da der Stab 24 hierbei vorgespannt wird, drückt letzterer dauerhaft auf das Ringelement 18. Das heißt gemäß den Fig. 19 bis 21 wird das Ringelement 18 nach oben gedrückt. Die Stifte 90, 92 sind nun so lang ausgeführt, dass diese aus dem Halteelement 32 herausragen und entsprechend der auf das Ringelement 18 wirkenden Kraft ebenfalls nach oben geneigt sind. Durch diese Neigung der Stifte 90, 92 werden die durch den Stab 24 auf die Stifte 90, 92 wirkenden Kräfte in einer Axial- und einer Radialkomponente zerlegt. Dabei bewirkt die Radialkomponente, dass die Haltestege 14, 16 nach innen gedrückt werden. Hierdurch wird gewährleistet, dass das Ringelement 18 auch bei großen, auftretenden Kräften zuverlässig in den Haltestegen 14, 16 fixiert wird.

In einer anderen, hier nicht dargestellten Ausführungsform sind die Stifte gekrümmt ausgebildet, ebenfalls um die Haltestege nach innen zu drücken.

Es versteht sich, dass die Flanke 80, 82 gemäß Fig. 16 bis 18 ebenfalls geneigt oder gekrümmt ausgebildet sein kann.

Der Stab 24 kann je nach Verwendungszweck beispielsweise als Verbindungs-, Distraktions- oder Kompressionsstab ausgebildet sein.

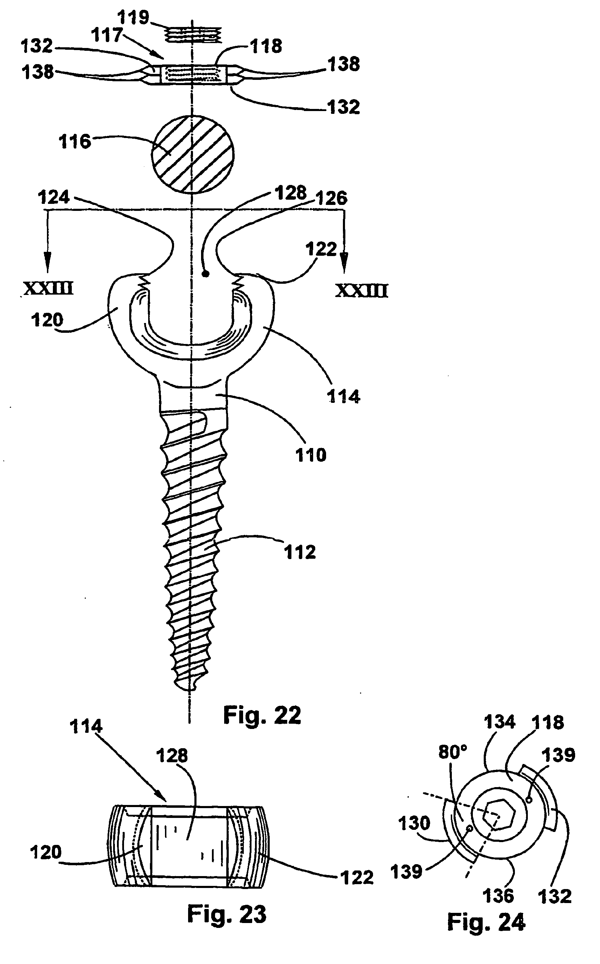

In den Fig. 22 - 24 ist ein weiteres Verankerungselement explosionsartig dargestellt. Dieses schraubenähnliche Verankerungselement 110 umfasst einen in den Wirbelknochen einschraubbaren Gewindeschaft, an dem eine Halterung 114 zur Befestigung eines Stabes 116 angebracht ist. Zum Verankerungselement 110 gehört auch ein Sicherungselement 117, welches ein Ringelement 118 und ein als Madenschraube ausgebildetes Feststellelement 119 umfasst. Das Sicherungselement 117 ist in die Halterung 114 einsetzbar und fixiert den Stab 116 in der Halterung 114.

Die Halterung 114 ist im Querschnitt U-förmig gebogen ausgeführt und umfasst zwei im Wesentlichen parallel angeordnete Haltestege 120, 122. Auf den sich jeweils zugewandten Seiten der Haltestege 120, 122 ist je ein Teilgewinde 124, 126 ausgebildet, welches als Sägezahngewinde ausgeführt ist.

Unter einem Sägezahngewinde sind neben dem metrischen Sägengewinde gemäß DIN 513 auch Sägezahngewinde mit einem etwas größeren oder etwas kleineren Flankenwinkel, mit einem Flankenwinkel von 0° oder einem negativen Flankenwinkel, sowie Sägezahngewinde gemäß EP 885 598 zu verstehen.

In der Halterung 114 ist zwischen den Haltestegen 120, 122 ein Aufnahmeschlitz 128 zur Aufnahme des Stabes 116 ausgebildet. Zur Fixierung dieses Stabes wird das Sicherungselement 117 mit seinem Ringelement 118 in die Teilgewinde 124, 126 eingesetzt und drückt somit den Stab 116 tief in den Aufnahmeschlitz 128 hinein und fixiert denselben.

Damit das Ringelement 118 zuverlässig in die Teilgewinde 124, 126 der Haltestege 120, 122 eingreifen kann, befinden sich auf dem Umfang des Ringelementes 118 zwei Gewindeabschnitte 130, 132. Wie in der Draufsicht gemäß Fig. 24 zu erkennen ist, liegen sich die Gewindeabschnitte 130, 132 genau gegenüber, während zwischen den jeweiligen Gewindeabschnitten 130, 132 Einführabschnitte 134, 136 ausgebildet sind. Das im Querschnitt kreisrunde Ringelement 118 hat außerhalb der Gewindeabschnitte 130, 132 einen Durchmesser von maximal dem Kerndurchmesser der Gewindeabschnitte 130, 132. Im Bereich der Gewindeabschnitte 130, 132 sind auf das Ringelement 118 jeweils zwei Gewindestege 138 aufgebracht, die den jeweiligen Gewindeabschnitt 130, 132 bilden. Analog zu den Teilgewinden 124, 126 sind auch die Gewindeabschnitte 130, 132 als Sägezahngewinde ausgeführt. Die Gewindeabschnitte 130, 132 erstrecken sich über ein Kreissegment von 80 ° und sind exakt gegenübenliegend angeordnet. Auf einer Oberseite des Ringelementes 118 sind zwei Werkzeugaufnahmen 139 vorgesehen, in die ein entsprechendes Werkzeug einsetzbar ist, mit dem das Ringelement 118 in die Halterung 114 einschraubbar ist. Im Ringelement 118 ist über ein Gewinde ein als Madenschraube ausgebildetes Feststellelement 119 gehalten, welches unabhängig vom Ringelement 118 einschraubbar ist, beispielsweise um den Stab endgültig zu fixieren. Dieses Feststellelement 119 besitzt eine Sechskantaufnahme 140, so dass das Feststellelement 119 mit einem Sechskant beliebig fest angezogen werden kann.

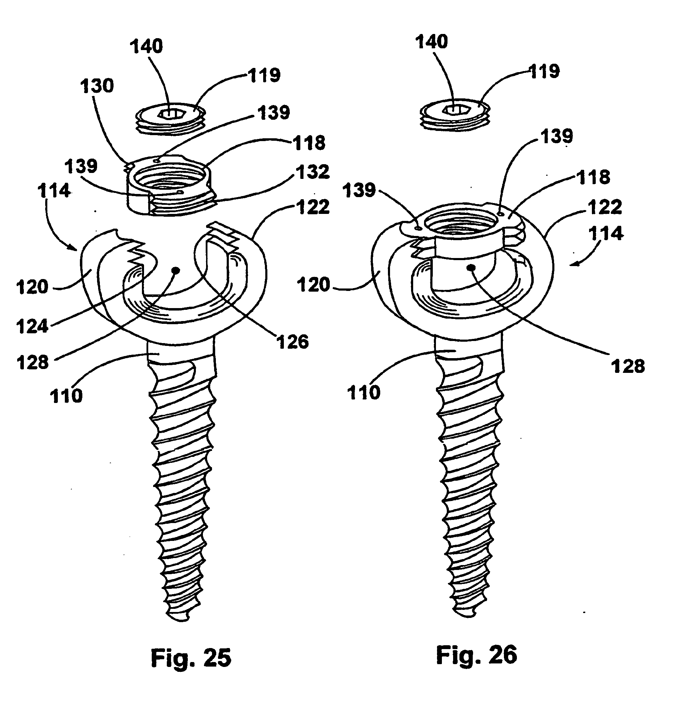

In den Fig. 25 und 26 ist das Verankerungselement 110 perspektivisch in zwei verschiedenen Positionen dargestellt, wobei Fig. 25 das Sicherungselement 117 oberhalb des eigentlichen Verankerungselementes 110 zeigt, während das Sicherungselement 117 in Fig. 26 in die Halterung 114 eingesetzt dargestellt ist. Zum Einsetzen des Sicherungselementes 117 wird das Ringelement 118 mit einer hier nicht dargestellten Zange oder einem ähnlichen Werkzeug ergriffen und anschließend axial in den Aufnahmeschlitz 128 der Halterung 114 eingeführt. Dabei ist das Ringelement 118 so ausgerichtet, dass dessen Einführabschnitte 134, 136 den Teilgewinden 124, 126 der Haltestegen 120, 122 gegenüberliegen, während die Gewindeabschnitte 130,132 in Richtung des Aufnahmeschlitzes 128 ausgerichtet sind und in dieser Position über die Halterung 114 hinausragen. Ist das Sicherungselement 118 tief genug in den Aufnahmeschlitz 128 eingeführt, so wird es um 90 ° verschwenkt. Nun werden die Gewindeabschnitte 130, 132 in das jeweilige Teilgewinde 124, 126 hineingeführt. In diesem Zustand fixiert das Sicherungselement 118 den Stab in der Halterung 114, wobei das Sägezahngewinde ein Auseinanderdrücken der Haltestege 120,122 verhindert und somit eine zuverlässige Befestigung des Stabes im Verankerungselement 110 gewährleistet.

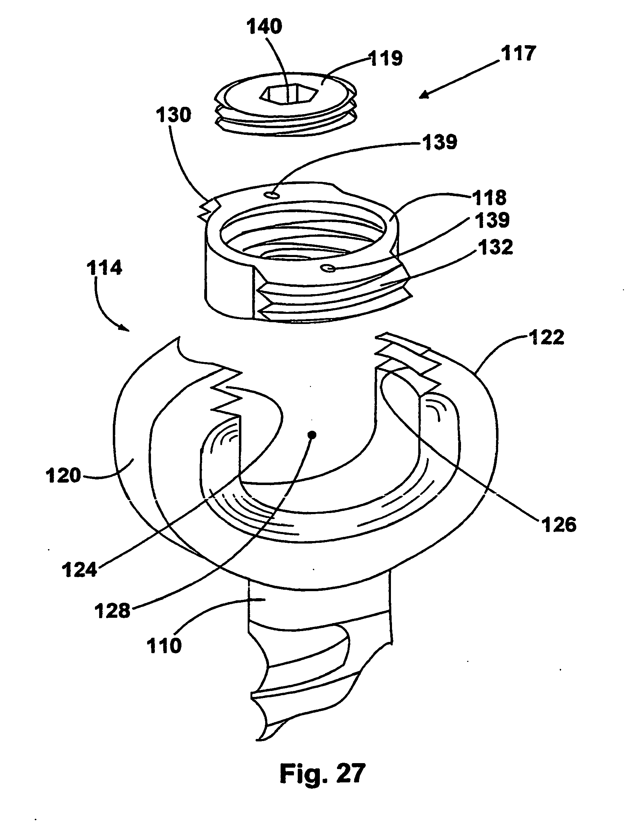

In der in Fig. 27 dargestellten Detailvergrößerung ist gut zu erkennen, dass die Gewindestege 138 der Gewindeabschnitte 130,132 an zumindest einer Stirnseite abgeflacht ausgebildet sind. Hierdurch wird das Einführen der Gewindeabschnitte 130, 132 in die Teilgewinde 124, 126 erleichtert, da die Gewindeabschnitte 130, 132 lediglich ungefähr in das Teilgewinde 124, 126 eingesetzt werden brauchen und sich ansonsten selbst in die richtige Position bewegen.

Durch das Reduzieren des Gewindes des Ringelementes 118 auf zwei sich gegenüberliegende Fast-Viertel-Gewinde ist es möglich, das Sicherungselement 117 im gewindefreien Bereich schnell und einfach an den Teilgewinden 124, 126 der Haltestege 120, 122 vorbeizuführen und durch eine 90 °-Drehung in diese einzuführen. Dies erleichtert die Arbeit des operierenden Chirurgen deutlich und führt zu einer signifikanten Verkürzung der Operationsdauer.

Der Stab 116 kann beispielsweise als Kompressions-, Verbindungs- oder Distraktionsstab ausgebildet sein, je nach Einsatzzweck. in einer anderen, hier nicht dargestellten Ausführungsform ist ein Teilgewinde 124 und/oder ein Teilgewinde 126 im Anschlag vorgesehen, so dass das Ringelement 118 nicht versehentlich zu weit gedehnt wird und wieder aus der Halterung 114 herausfällt.

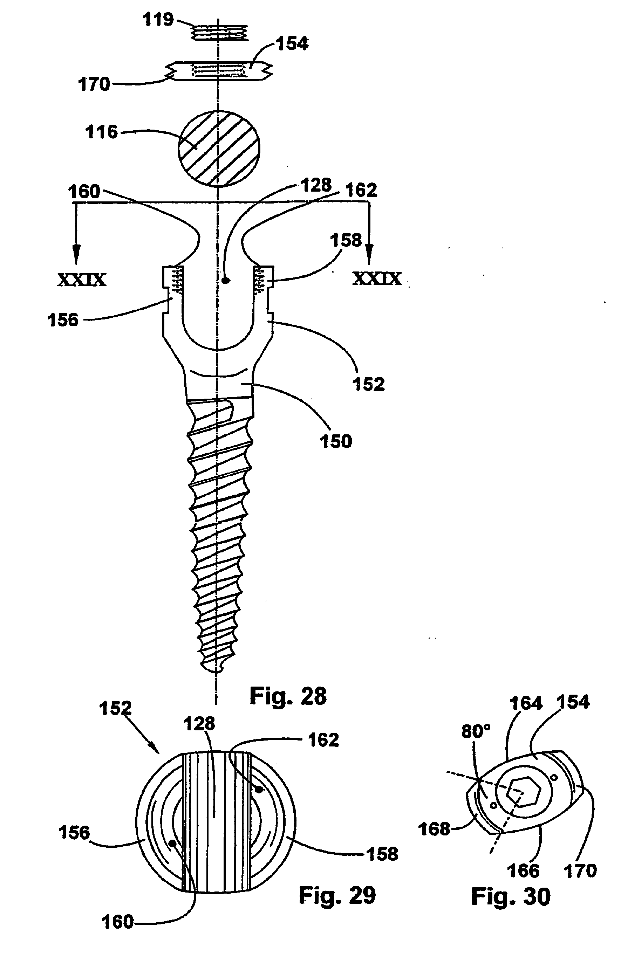

In den Figuren 28 bis 30 ist noch ein weiteres Verankerungselement 150 dargestellt, welches sich durch die Ausgestaltung der Halterung 152 und ein dementsprechend angepasstes Ringelement 154 von der ersten Ausführungsform unterscheidet. Im Unterschied zur ersten Ausführungsform sind die Haltestege 156, 158 der Halterung 152 breiter dimensioniert, als die in den Haltestegen 156, 158 angebrachten Teilgewinde 160, 162, so dass die Teilgewinde 160, 162 nicht bis an den jeweiligen Rand der Haltestege 156, 158 reichen, sondern von diesen beabstandet enden.

Wie aus Figur 30 besonders gut hervorgeht, ist das Ringelement 154 an seinen Einführabschnitten 164 und 166 mit einer vergleichsweise glatten und konvex gewölbten Oberfläche ausgebildet, um in einfacher Weise in den Aufnahmeschlitz 128 zwischen den Haltestegen 156,158 eingeführt und dort verdreht zu werden, bis die Gewindeabschnitte 168,170 in die jeweiligen Teilgewinde 160, 162 eingreifen, und das Ringelement 154 zuverlässig halten.

### Bezugszeichenliste:

- 10: Gewindeschaft
- 12: Halterung
- 14: Haltesteg
- 16: Haltesteg
- 18: Ringelement
- 20: Feststellelement
- 22: Aufnahmeschlitz
- 24: Stab
- 26: Stift
- 28: Stift
- 30: Einführung
- 32: Halteelement
- 34: Innengewinde
- 40: Gewindeschaft
- 42: Halterung
- 44: Haltesteg
- 46: Haltesteg
- 48: Ringelement
- 50: Feststellelement
- 52: Innengewinde
- 54: Aufnahmeschlitz
- 56: Stift
- 58: Stift
- 61: Einführung
- 62: Halteelement
- 63: Halteelement
- 66: Halteelement
- 68: Verbreiterung
- 70: Halteelement
- 72: Flanke
- 74: Flanke
- 76: Einführung
- 77: Einführung
- 78: Halteelement
- 80: Flanke
- 82: Flanke
- 84: Einführung
- 86: Halteelement
- 90: Stift
- 92: Stift
- 110: Verankerungselement
- 112: Gewindeschaft
- 114: Halterung
- 116: Distraktions- oder Kompressionsstab
- 117: Sicherungselement
- 118: Ringelement
- 119: Feststellelement
- 120: Haltesteg
- 122: Haltesteg
- 124: Teilgewinde
- 126: Teilgewinde
- 128: Aufnahmeschlitz
- 130: Gewindeabschnitt
- 132: Gewindeabschnitt
- 134: Einfünrabschnitt
- 136: Einführabschnitt
- 138: Gewindesteg
- 139: Werkzeugaufnahme
- 140: Sechskantaufnahme
- 150: Verankerungselement
- 152: Halterung
- 154: Ringelement
- 156: Haltesteg
- 158: Haltesteg
- 160: Teilgewinde
- 162: Teilgewinde
- 164: Einführungsabschnitt
- 166: Einführungsabschnitt
- 168: Gewindeabschnitt
- 170: Gewindeabschnitt

## Patentansprüche

1. Verankerungselement zur Befestigung eines Stabes einer Vorrichtung zum Einrichten einer menschlichen oder tierischen Wirbelsäule an einem Wirbelknochen, mit einer im wesentlichen U-förmig ausgebildeten und zwei im wesentlichen parallel angeordnete Haltestege (14, 16, 44, 46) umfassenden Halterung (12, 42), in dem ein Aufnahmeschlitz (22, 54) zur Aufnahme des Stabes (24) ausgebildet ist, und mit einem gegen den im Aufnahmeschlitz (22, 54) aufgenommenen Stab (24) wirkenden Sicherungselement, wobei das Sicherungselement ein Feststellelement (20, 50) und ein Ringelement (18, 48) umfasst, wobei das Ringelement (18, 48) mittels einer Renkverbindung am freien Ende der Haltestege (14, 16, 44, 46) anbringbar ist, wobei die Renkverbindung als Bajonettverschluss ausgebildet ist, wobei der Bajonettverschluss eine schlitz- oder nutartige Aufnahme und einen in die Aufnahme eingreifenden Stift (90, 92) umfasst,
**dadurch gekennzeichnet,**
**dass** der im wesentlichen radial abstehende Stift (90, 92) durch die Aufnahme hindurch und aus dieser heraus reicht, wobei der Stift (90, 92) derart gekrümmt oder geneigt ist, dass der im Bereich des Halteelementes (32) am Haltesteg (14, 16) zur Anlage kommende Stift (90, 92) diesen nach innen drückt.

2. Verankerungselement nach Anspruch 1,
**dadurch gekennzeichnet,**
**dass** der Stift (90, 92) in Richtung der auf das Sicherungselement wirkenden Kräfte gekrümmt oder geneigt ist.

3. Verankerungselement nach einem der voranstehenden Ansprüche,
**dadurch gekennzeichnet,**
**dass** die Aufnahme eine axiale Einführung (30, 61, 76, 77, 84) und ein tangentiales Halteelement (32, 62,63, 66, 70, 78, 86) umfasst.

4. Verankerungselement nach Anspruch 3,
**dadurch gekennzeichnet,**
**dass** die Einführung (30) V-förmig ausgebildet ist.

5. Verankerungselement nach wenigstens einem der Ansprüche 3 bis 4,
**dadurch gekennzeichnet,**
**dass** das Halteelement (66, 70) leicht nach oben steigend ausgebildet ist.

6. Verankerungselement nach wenigstens einem der Ansprüche 3 bis 5,
**dadurch gekennzeichnet,**
**dass** sich das Halteelement (66) am Ende verbreitert.

## Claims

1. An anchoring element for fastening a rod of a device for adjusting a human or animal spine to a vertebral bone, said anchoring element having a holding device (12, 42) that is substantially U-shaped and includes two substantially parallely disposed holding ridges (14, 16, 44, 46), a rod (24) receiving slot (22, 54) being formed therein, and a securing element acting against the rod (24) accommodated in the receiving slot (22, 54), said securing element including a locking element (20, 50) and a ring element (18, 48), said ring element (18, 48) being mountable to the free end of the holding ridges (14, 16, 44, 46) by means of a single turn coupling system, said single turn coupling system being configured to be a bayonet coupling, said bayonet coupling including a slot-type or a groove-type receiving recess and a pin (90, 92) engaging in said receiving recess,
**characterized in**
**that** the substantially radially projecting pin (90, 92) extends through the receiving recess and protrudes therefrom, said pin (90, 92) being bent or inclined so that the pin (90, 92) abutting on the holding ridge (14, 16) in the region of the holding element (32) pushes said holding ridge inward.

2. The anchoring element as set forth in claim 1,
**characterized in**
**that** the pin (90, 92) is bent or inclined in the direction of the forces acting onto the securing element.

3. The anchoring element according to one of the aforesaid claims,
**characterized in**
**that** the receiving recess includes an axial entrance (30, 61, 76, 77, 84) and a tangential holding element (32, 62, 63, 66, 70, 78, 86).

4. The anchoring element as set forth in claim 3,
**characterized in**
**that** the entrance (30) is of a V-shape configuration type.

5. The anchoring element as set forth in at least one of the claims 3 to 4,
**characterized in**
**that** the holding element (66, 70) is configured to slightly extend upward.

6. The anchoring element as set forth in at least one of the claims 3 through 5,
**characterized in**
**that** the holding element (66) widens at its end.

## Revendications

1. Elément d'ancrage pour fixer une tige d'un dispositif de réduction d'une colonne vertébrale humaine ou animale sur une vertèbre, avec une fixation (12, 42) sensiblement en U et comprenant deux branches de retenue (14, 16, 44, 46) disposées sensiblement parallèlement entre elles, une fente (22, 54) de réception de la tige (24) étant pratiquée dans cet élément d'ancrage qui possède également un élément de sécurisation agissant contre la tige (24) logée dans la fente (22, 54) de réception, cet élément de sécurisation comprenant un élément d'arrêt (20, 50) et un élément annulaire (18, 48), l'élément annulaire (18, 48) étant apte à être monté sur l'extrémité libre des branches de retenue (14, 16, 44, 46) au moyen d'une liaison à crans, cette liaison à crans étant réalisée sous forme de fermeture à baïonnette, cette fermeture à baïonnette comprenant un logement du type fente ou rainure et un ergot (90, 92) s'engageant dans le logement,
**caractérisé en ce**
**que** l'ergot (90, 92) en saillie sensiblement radiale traverse le logement et en dépasse, cet ergot (90, 92) étant incurvé ou incliné de manière à ce que l'ergot (90, 92) arrivant en butée sur la branche de retenue (14, 16) à la hauteur de l'élément de retenue (32) pousse celui-ci vers l'intérieur.

2. Elément d'ancrage selon la revendication 1,
**caractérisé en ce**
**que** l'ergot (90, 92) est incurvé ou incliné dans la direction des forces agissant sur l'élément de sécurisation.

3. Elément d'ancrage selon l'une quelconque des revendications précédentes,
**caractérisé en ce**
**que** le logement comprend une insertion axiale (30, 61, 76, 77, 84) et un élément de retenue tangentiel (32, 62, 63, 66, 70, 78, 86).

4. Elément d'ancrage selon la revendication 3,
**caractérisé en ce**
**que** l'insertion (30) est conformée en V.

5. Elément d'ancrage selon une au moins des revendications 3 à 4,
**caractérisé en ce**
**que** l'élément de retenue (66, 70) est réalisé de manière à s'étendre légèrement vers le haut.

6. Elément d'ancrage selon une au moins des revendications 3 à 5,
**caractérisé en ce**
**que** l'élément de retenue (66) s'élargit à son extrémité.
